Europäisches Patentamt

European Patent Office

Office européen des brevets

(11) Veröffentlichungsnummer: **0 436 834 A2**

## (12) EUROPÄISCHE PATENTANMELDUNG

(21) Anmeldenummer: 90123262.9

(22) Anmeldetag: **05.12.90**

(51) Int. Cl.⁵: **A61K 6/04, C23C 24/10**

(30) Priorität: **08.01.90 DE 4000302**

(43) Veröffentlichungstag der Anmeldung:
**17.07.91 Patentblatt 91/29**

(84) Benannte Vertragsstaaten:
**AT BE CH DE DK ES FR GB GR IT LI LU NL SE**

(71) Anmelder: **Degussa Aktiengesellschaft
Weissfrauenstrasse 9
W-6000 Frankfurt am Main 1(DE)**

(72) Erfinder: **Schiwiora, Harry
Theodor-Heuss-Strasse 37
W-7530 Pforzheim(DE)**
Erfinder: **Stümke, Manfred, Dr. Dipl.-Phys.
Am Waldweg 21
W-7530 Pforzheim(DE)**

(54) **Goldhaltiges Präparat zur Herstellung von nochporösen Beschichtungen.**

(57) Zur Herstellung hochporöser Beschichtungen auf Metalloberflächen durch Aufsintern verwendet man bekannte Goldpräparate und setzt diesen 1 bis 10 Gew. % eines Porenbildners zu, der aus einem Material besteht, das bei 700 bis 1050° C schlagartig sich zersetzt oder verdampft und rückstandsfrei aus der Schicht entweicht. Vorzugsweise verwendet man hierfür Kunststoffe und Dentalwachse in Pulverform.

EP 0 436 834 A2

## GOLDHALTIGES PRÄPARAT ZUR HERSTELLUNG VON HOCHPORÖSEN BESCHICHTUNGEN

Die Erfindung betrifft ein goldhaltiges Präparat zur Herstellung von duktilen, hochporösen Beschichtungen auf Metalloberflächen durch Aufsintern bei 700 bis 1050 ° C, insbesondere zum Überziehen metallischer Teile in der Dentaltechnik, bestehend aus 1 bis 10 Gew. % eines Porenbildners und 90 bis 99 Gew. % einer Mischung, die sich zusammensetzt aus 50 bis 95 Gew. % Goldpulver und 5 bis 50 Gew. % organischem Binde- und Lösungsmittel und gegebenenfalls Zusätzen von Metall- und/oder Keramikpulvern.

Beim Verbund von metallischen Teilen mit solchen aus anorganischen oder organischen Materialien spielen die Gestaltung und die Verformbarkeit der Grenzflächen für die Güte der Haftung eine wichtige Rolle. Für eine innige mechanische Verzahnung von Unterlage und Beschichtung wird ein geeignetes Oberflächenprofil mit einem hohen Anteil an Unterschnitten angestrebt; für Klemmsitz bzw. zum Abbau mechanischer Spannungen in der Verbindungszone sind kompressible bzw. duktile Zwischenschichten vorteilhaft. Da ein verbesserter Werkstoffverbund sich direkt in einer höheren Gebrauchssicherheit bzw. in einer längeren Gebrauchsdauer auswirkt, ist ein Bedarf an derartig präparierten Grenzflächen insbesondere in der Dentaltechnik gegeben, z.B. zur Erzielung einer komprimierbaren Beschichtung oder zur Ausbildung einer infiltrierbaren Zwischenschicht für den Verbund mit anderen Materialien zum Präparieren der Oberfläche von metallischen Teilen, die spannungsarm eingepaßt oder verklebt oder mit Kunststoff verblendet werden sollen.

Beispielsweise wird in der DE-OS 37 41 847 ein Wurzelstift beschrieben, der mit einer porösen. zusammenpreßbaren Schicht aus Feingold versehen ist, die sich beim Einsetzen des Stiftes mit definiertem Druck durch plastische Verformung verdichten und dadurch einen druckarmen, sehr engen Kontakt zu den Wurzelwänden herstellen kann.

Bei der Verblendung von Zahnersatzgerüsten aus Aufbrennlegierungen mit dentalkeramischen Massen kommen vorteilhafterweise goldhaltige Zwischenschichtmaterialien zur Anwendung, wie sie z.B. in den DE-OSen 28 51 729, 30 27 472 und 30 27 473 beschrieben werden. Die sich bei der Anwendung dieser Präparate in den Zwischenschichten ergebende Porosität reicht im allgemeinen für eine mechanische Verzahnung der Deckschichten und zur Spannungspufferung aus. In Einzelfällen ist jedoch eine noch weiterreichende Verzahnung von Zwischenschichtmaterial und Verblendkeramik notwendig.

Bei der in der Dentaltechnik ebenfalls angewendeten Verblendung von Zahnersatzgerüsten mit Kunststoffen stellt die Spalt- und Rißbildung zwischen dem Verblendkunststoff und dem metallischen Gerüst ein grundsätzliches Problem dar, bei dem die obigen Präparate von der Struktur her keine geeigneten Zwischenschichten liefern.

Neben mannigfaltigen Vorschlägen, beispielsweise Aufbringung von Makro- oder Mikroretentionen, Beschichtung mit Silanen, Siliziumdioxid oder Zinn, ist in dem DE-GM 82 07 485 ein Goldpräparat beschrieben, dem Keramikteilchen zugemischt werden. Nach dem Aufbrennen zwischen 950 ° C und 1100 ° C wird der Keramikanteil anschließend mit Flußsäure wieder herausgelöst. Nachteilig hierbei sind die hohe Sintertemperatur, die für die üblicherweise für Kunststoffverblendung verwendeten Legierungen nicht zuträglich ist. sowie die zum Herauslösen der oxidkeramischen Bestandteile notwendige Verwendung von Flußsäure, deren gesundheitsschädliche Wirkung bekannt ist.

Ähnliche Probleme wie beim Metall-Kunststoff-Verbund liegen auch in der Klebebrückentechnik vor. Auch hier führt eine verbesserte Mikroretention der Kleberschicht am Metallgerüst zu einer Verminderung der Versagensquote. Während chemische oder elektrochemische Ätzungen nur bei wenigen heterogenen Nichtedelmetall-Legierungen zu geeigneten Oberflächen-Mikrostrukturen führen, macht eine retentive Zwischenschicht auf homogenen und hochkorrosionsbeständigen Edelmetall-Legierungen auch diese für die Klebebrückentechnik verwendbar.

Da für die meisten Anwendungsfälle nur solche Zwischenschichtmaterialien in Frage kommen, die selbst eine hohe Korrosionsbeständigkeit aufweisen, bietet sich Gold als Hauptkomponente eines solchen Materials an.

Es war daher Aufgabe der vorliegenden Erfindung, ein goldhaltiges Präparat zur Herstellung von duktilen, hochporösen Beschichtungen auf Metalloberflächen durch Aufsintern bei 700 bis 1050 ° C zu entwickeln, insbesondere zum Überziehen metallischer Teile in der Dentaltechnik, bestehend aus 1 bis 10 Gew. % eines Porenbildners und 90 bis 99 Gew. % einer Mischung, die sich zusammensetzt aus 50 bis 95 Gew. % Goldpulver und 5 bis 50 Gew. % organischen Binde- und Lösungsmitteln und gegebenenfalls Zusätzen von Metall- und/oder Keramikpulvern, die eine hohe Haftfestigkeit auch für Kunststoffe ergeben.

Diese Aufgabe wird erfindungsgemäß dadurch gelöst, daß als Porenbildner ein pulverförmiges Material eingesetzt wird, das bei der Sintertemperatur schlagartig sich zersetzt oder verdampft und rückstandsfrei entweicht.

Man geht dabei von bekannten Präparaten aus, wie sie in den DE-OS en 30 27 472 und 30 27 473 beschrieben sind, und setzt 90 bis 99 Gew. % dieser Präparate 1 bis 10 Gew. % des Porenbildners zu. Vorzugsweise verwendet man hierfür ein Kunststoff- und/oder Wachspulver, das bei 700 bis 850° C innerhalb von wenigen Millisekunden schlagartig verdampft oder sich zersetzt und im Zeitraum von 2 bis 5 Minuten rückstandsfrei aus der Schicht entweicht. Besonders geeignet sind Pulver mit einer maximalen Teilchengröße von 1 mm, wobei es sich als vorteilhaft erwiesen hat, zwei Fraktionen von Pulvern zu verwenden, wobei 50 bis 99 Gew. % eine Größe bis zu 350 um aufweisen und 1 bis 50 Gew. % eine Größe zwischen 350 und 800 um. Besonders vorteilhaft ist der Zusatz von 65 bis 75 Gew. % Pulverteilchen bis 350 um und 25 bis 35 Gew. % Pulverteilchen zwischen 350 und 700 um. Weiterhin ist es günstig, kugelige Teilchen zu verwenden und 3 bis 7 Gew. % an Porenbildnerteilchen zuzusetzen.

Als Kunststoffpulver können beispielsweise Polymethylmethacrylatteilchen und als Wachspulver Dentalwachsteilchen gemäß DIN 13 908 verwendet werden.

Beispielsweise wird eine Mischung von 100 Gewichtsteilen eines Präparates gemäß DE-OS 30 27 473 mit 5 Gewichtsteilen eines Dentalwachspulvers gemäß DIN 13 908, bestehend aus 70 % Kugeln mit Durchmessern bis zu 350 um und 30 % Kugeln mit Durchmessern zwischen 350 und 700 um, auf Teile aus Gold-Palladium- und Silber-Palladium-basislegierungen bei 700 bis 850° C während 2 bis 5 Minuten aufgesintert. Es ergeben sich je nach Auftrag zusammenhängende Beschichtungen von 25 bis 350 um Dicke mit einem hohen Anteil überwiegend offener Poren, ohne erkennbaren Einfluß von der Art der als Substrat verwendeten Legierung.

So hergestellte retentive Goldbeschichtungen lassen sich gut und dauerhaft nicht nur mit Verblendkeramik, sondern insbesondere auch mit Verblendkunststoff verbinden. Der sich hierbei ergebende Verbund hat überraschenderweise eine so hohe Stabilität und Rißfestigkeit, daß bei einseitigen Kunststoffverblendungen von Legierungsplättchen diese infolge der Polymerisationsschrumpfung des Kunststoffs gekrümmt werden und sich dieser nicht, wie sonst üblich, von den Plättchen abzieht. Dieser unerwartet stabile Verbund ist auch nach mehrwöchiger Lagerung in entionisiertem Wasser bei Raumtemperatur noch intakt, so daß eine wesentliche Verlängerung der Gebrauchsdauer erreicht wird.

**Patentansprüche**

1. Goldhaltiges Präparat zur Herstellung von duktilen, hochporösen Beschichtungen auf Metalloberflächen durch Aufsintern bei 700 bis 1050° C, insbesondere zum Überziehen metallischer Teile in der Dentaltechnik, bestehend aus 1 bis 10 Gew. % eines Porenbildners und 90 bis 99 Gew. % einer Mischung, die sich zusammensetzt aus 50 bis 95 Gew. % Goldpulver und 5 bis 50 Gew. % organischem Bindemittel und Lösungsmittel und gegebenenfalls Zusätzen von Metall- und/oder Keramikpulvern,
dadurch gekennzeichnet,
daß als Porenbildner ein pulverförmiges Material eingesetzt wird, das bei der Sintertemperatur schlagartig sich zersetzt oder verdampft und rückstandsfrei entweicht.

2. Goldhaltiges Präparat nach Anspruch 1
dadurch gekennzeichnet,
daß als Porenbildner ein Kunststoff- und/oder Wachspulver eingesetzt wird, das bei 700° bis 850° C sich schlagartig zersetzt oder verdampft.

3. Goldhaltiges Präparat nach Anspruch 1 und 2,
dadurch gekennzeichnet,
daß Pulver mit einer Teilchengröße von maximal 1 mm Durchmesser eingesetzt werden.

4. Goldhaltiges Präparat nach Anspruch 1 bis 3,
dadurch gekennzeichnet,
daß 50 bis 99 Gew. % des Pulvers eine Teilchengröße bis 350 um und 1 bis 50 Gew. % eine Teilchengröße zwischen 350 und 800 um aufweisen.

5. Goldhaltiges Präparat nach Anspruch 1 bis 4,
dadurch gekennzeichnet,
daß 65 bis 75 Gew. % der Pulverteilchen eine Größe bis 350 um und 25 bis 35 Gew. % eine Größe von 350 bis 700 um aufweisen.

6. Goldhaltiges Präparat nach Anspruch 1 bis 5,
dadurch gekennzeichnet,
daß die Pulverteilchen eine kugelige Form aufweisen.

7. Goldhaltiges Präparat nach Anspruch 1 bis 6,
dadurch gekennzeichnet.
daß die porenbildenden Pulver in Mengen von 3 bis 7 Gew. % zugemischt sind.

8. Verwendung von goldhaltigen Präparaten nach Anspruch 1 bis 7 zur Herstellung von duktilen, hochporösen Beschichtungen auf Teilen in der Dentaltechnik.